# EUROPEAN PATENT APPLICATION

(11) **EP 2 762 171 A1**
(43) Date of publication of application: **06.08.2014**
(21) Application number: 13153503.1
(22) Date of filing: 31.01.2013
(51) Int. Cl.: A61L 24/00, C08G 83/00, C09J 201/00

(54) **Polymer adhesive**

(71) Applicant: National University of Ireland, Galway, Galway (IE)
(72) Inventor: Wang, Dr. Wenxin, Galway (IE); Zheng, Yu, University of Cambridge, CB2 1EW (GB); Zhang, Hong, Galway (IE); Pandit, Abhay, Galway (IE); DaCosta, Mark, Galway (IE); Bre, Ligia Pereira, Galway (IE)
(74) Representative: Gates, Marie Christina Esther

(57) **Abstract**

A dendritic or hyperbranched polymer comprising a plurality of branches, wherein at least one of the branches comprises at least one catechol moiety that is oxidisible to a semiquinone moiety and/or a quinone moiety.

## Description

### Field of the Invention

The invention relates to the provision of biocompatible and biodegradable tissue adhesives which are based on dendritic/hyperbranched polymers. The tissue adhesives of the invention may be used as a wet tissue adhesive, for example, as a sternum bone closure adhesive, an incisional or laceration wound closure adhesive.

### Background to the Invention

Wound closure and healing are essential for achieving optimal outcomes for all surgical patients. The healing process is facilitated by proper closure of the surgical wound. Traditionally, wound healing has been a particularly invasive practice. Devices such as sutures and staples have been used for many years. These methods can be traumatic to the patient. The ideal incision closure should be simple, effective, safe, rapid, inexpensive, painless, cosmetic and bactericidal. Although there has been great research into mechanical methods there is still the possibility of localised infection and inflammation of the wound area. Infection can arise from the presence of an external body physically carrying antibiotic - resistant bacteria detrimental to the patient's health. In general, the use of tissue adhesives can significantly decrease health-care costs and is preferred by patients. Adhesives provide a needle-free method of wound closure, an important consideration in light of the risks presented by blood-borne viruses. In addition, adhesives do not require local anaesthetics.

With a fast curing adhesive, the wound can be closed quickly and it wears off after four or five days, which makes the process simpler and better. The topical skin adhesive adheres to the skin and itself, forming a clean strong adherent bond that holds the edges of skin wounds together so that the wounds can heal normally underneath the film. When intact, the film acts as a microbial barrier to protect the wound from potential contamination. Topical tissue adhesives slough from the skin in five to ten days as the skin heals underneath and it is no longer required, thus eliminating the need for non-absorbable suture or staple removal. Current adhesives available in the market for wound healing can be categorized into three groups: fibrin glue, synthetic polymer adhesive and biomimetic polymer adhesive. Fibrin sealants or "fibrin glues," which are the end product of the coagulation cascade, serve as a primary hemostatic plug as well as a matrix to enhance wound healing. In contrast to synthetic adhesives, fibrin sealants have the advantage of being biocompatible and biodegradable. The fibrin clot is resorbed within days to weeks as a part of the normal wound-healing process. As such, they are not associated with inflammation, foreign body reactions, tissue necrosis, or extensive fibrosis. However, fibrin glues widely used in surgical practice do not provide significant adhesive strength, optimal application requires dry substrates, and is high cost and associated with risk of viral transmission.

Two mainly synthetic polymer adhesive available are cyanoacrylates and polyethylene glycol based products. Topical cyanoacrylate skin adhesives offer many advantages over traditional wound closure devices. The cyanoacrylates function as waterproof occlusive dressings, have microbial barrier properties against gram-positive organisms, and may also reduce infections. Topical skin adhesives, therefore, provide not only primary closure but an effective microbial barrier throughout this critical time period. Cyanoacrylates are synthetic glues that rapidly polymerize on contact with water or blood. n-Butyl-cyanoacrylates have shorter molecular chain lengths, this allows the product to require only one layer and dry significantly faster while still maintaining its efficacy and strength. These topical skin adhesives dry in 30 seconds, allow for a one-layer application, and have a low exothermic reaction. 2-octyl cyanoacrylate (Dermabond®) have longer molecular chain lengths, this allows the product to have more flexibility and smooth appearance during healing while still maintaining its efficacy and strength. They do however take a longer time to dry (polymerize) as a result of the two layer application and longer molecular chain lengths. 2-octyl cyanoacrylate degrades much more slowly due to its longer organic backbone which slows the degradation of the adhesive enough to remain below the threshold of tissue toxicity. Due to the toxicity issues of cyanoacrylate, the use of 2-octyl cyanoacrylate for sutures is preferred. Cyanoacrylate adhesives appear to have greater performance in external tissue bonding applications, particularly in medical emergency, but it is moisture sensitive and toxic to the exposed wound site.

Polyethylene glycol (PEG) polymers are hydrogels that are used for tissue adhesion. FocalSeal-L is a water-soluble, bioabsorbable compound that requires a significant amount of time for photoactivation, which is a limitation for its use in hemostasis. Another PEG polymer, CoSeal is the first PEG-based sealant approved by FDA for use in the US, does not require the same activation source and is currently being used in Europe for similar purposes. FocalSeal has thus far proved useful in decreasing air leaks after major thoracic surgery but has not yet been used in the GI endoscopic domain. Coseal allows surgeons to operate vascular-related surgeries with improved safety and efficiency. However, the polyethylene glycol (PEG) glues have the disadvantages of being applied as a two-step process and some still require UV photo-crosslinking. Recently, lots of researches have been done work on this part, for instance, acrylic adhesives, hydrocolloid adhesives, hydrogel adhesives, polyurethane adhesives and soft silicone adhesives, but most of them have to be used on wound dressings and regrettably, none of them is a perfect tissue adhesive for internal wound repair.

Biomimetic adhesives are seen as an alternative to biological adhesives. Biomimetic adhesives are designed to mimic the crosslinking process that occurs naturally in blood clotting. There has been increased interest and research into biomimetic adhesives as they have shown the appealing adhesive properties in wet environments. However, there are currently no biomimetic skin adhesive products on the market today.

A median sternotomy the most commonly performed procedure in cardiothoracic surgery with over 1.4 million performed annually worldwide. This procedure is performed by separating the tissue over the sternum with a scalpel and then dividing the sternum with an electric saw. During the operation the sternum is held open with a sternal retractor. After the procedure the sternum must be held together to allow for healing. The quality with which the sternum is held together is of vital important to the healing process as it affects pain, the possibility of morbidity and mortality, healing time and the strength of the bone when repaired. The standard methods for closing this wound use stainless steel wire and pins which pierce the bone. However there are a number of associated problems. Aside from the discomfort endured by patients who use stainless steel wire, there is an associated morbidity of 0.5-8% and mortality rate 10-40% with this technique. These methods are not biodegradable and require a second surgery for removal which increases the likelihood of surgical complications, they are traumatic and cause damage to the bone and can delay emergency surgery in the case of complications which is most likely to be necessary in the first 24 hours after the surgery. The first method of closure is to pierce the bone, and suture the bone closed using stainless steel wire. This is normally done using 6 wires. This is the standard of care despite evidence of pathologic sternal displacement (>2 mm) during physiologic distracting forces (coughing). Unstable median sternotomy closure can lead to significant post-operative morbidity. Functional recovery, respiration, pain, sternal dehiscence and infection are all influenced by post-operative sternal stability. The use of this method severely reduces patient's mobility and strength for eight to twelve weeks following the surgery. After this period patients still need to be extremely careful. A more efficient method to replace this invasive and impractical method of closure has long been sought by doctors. Research recently published by Global Analysts Inc placed the future market potential for wound closure technologies at $2.08 billion by 2015. The same research also showed that skin adhesives and sealants have experienced a 10-20% growth rate in the past two years. Also the bone cement and accessory market is directly related to the number of hip, knee, shoulder, elbow, digit, ankle, and wrist arthroplasty procedure performed annually. Due to better living conditions and improvements in medical care the average global age is always increasing. Because of this it is predicted that the number of patients requiring bone cement treatments will increase driving up the bone cement market. The US market for orthopaedic biomaterials, of which bone cements are a part, is expected to grow by 12% per annum over the next four years.

Adhesives, especially bone adhesives, are resorbed and degraded to non-toxic products after fulfilling their function in contact with the living organism. The use of such bone adhesives has found growing interest in all fields of medicine in the last 50 years. The first bone adhesive was described in 1931 by Hedri and was a mixture of collagen and fibrous protein 'ossocol'. Good initial bonding strength and healing of fractures were reported, but severe allergic reactions prevented its use. Afterwards, a lot of researches were carried out on epoxy resins, cyanoacrylates, polyurethanes, polymethylmethacrylates (PMMA), fibrin adhesives, gelatin-resorcin-aldehydes, protein-aldehyde systems, peptide-based adhesives, adhesives based on oligo- and polylactone and alkylenebis(oligolactoyl)-methacrylates. In spite of the large amount of work which has been done in the last decade on tissue and bone adhesives, it is evident that the development of a successful bone adhesive is in its very early stages. Nowadays, widely used biodegradable materials as bone adhesives include PGA, PLLA, PDLLA, PGA/TMC, PDS and PBHBA. Research studies reveal that they are safe for majority of ankle fractures, but not suitable for comminuted and unstable fractures and most bone adhesives being used now are cements with minimal adhesive properties, which bond surfaces together by interlocking into pores and imperfections in the surfaces of the underlying bone. Canadian researchers have pioneered a new bone cement, called Kryptonite®, a biocompatible polymer, which is widely used in healing bone damage. This bone cement has also been used to aid repair in a number of median sternotomy cases. It has been shown to provide an improved sternal stability over using wires alone, which will improve healing time and comfort. It also allows for increased mobility shortly following surgery. KRYPTONITE^{™} which allows the breast bone to adhere together within hours instead of several weeks as is the case when using traditional methods, is the first adhesive which have found a way to help open heart surgery patients to recover better and faster. It is suggested that the use of adhesive in chest surgery increased mechanical strength of the breastbone closure by 5 to 10 times that of wires alone, reduces the normal recovery time by half and patients have less physical disability in the first six weeks after surgery and can breathe deeply sooner. Patients are able to cough with less discomfort and require significantly less medication such as narcotics to manage pain. However the bonding strength in the first 24 hours is too high to allow for easy emergency surgery. Also post operative complications often occur shortly after the initial procedure requiring the chest to be reopened in which case the steel wire or pins could cause fatal delay. As well as of this a non biodegradable method of sternum bone closure will require a second surgery which will increase the likelihood of fatal harm. An alternative to this method that can reduce discomfort, healing time and allow for easy access within 24 hours is needed. The aim of this project is to develop a biodegradable bone adhesive tailored for this application. Recently, more research has been carried out to develop adhesives for effective bone repair. Biodegradable bone adhesives are materials that induce complete bone regeneration and disappear as soon as the bone recovers, since they are gradually self-degrading in the body. Biodegradable adhesives thus serve as an alternative to conventional adhesives and have the advantage of not being necessary to remove once the fracture has healed. KRYPTONITE^{™} Bone Cement represents a major advance in treating cranial defects due to trauma and surgery and it is the first non-toxic, low exotherm cement and bone void filler approved for cranioplasty applications that offers strong adhesive properties to organic and inorganic materials. KRYPTONITE^{™} Bone Cement is a selfsetting bone cement formed by combining three naturally occurring components to create an adhesive, non-toxic bone repair solution that cures with porous properties and is engineered to mimic natural bone. However, as mentioned above, KRYPTONITE^{™} should not be used in sternal closure cases, since its crosslinking speed is too high. Within just six minutes it will reach high adhesion strength. It has been used in some cases and provides an improvement over steel wire and pins, however a more appropriate alternative is still needed. The high strength which the cement reaches within a short time means it is not recommended for patients at risk of internal bleeding or other post surgical complications as use of this adhesive would delay the emergency surgery. These complications are likely to arise within 24 hours of the operation. Also this bone cement is used in conjunction with the traditional method of stainless steel wires, which still requires a second surgery for removal.

### Object of the Invention

It is an object of the present invention to provide a bio-compatible topical skin adhesive with the following qualities: good biocompatibility, good wet adhesion, relatively strong strength within 15 mins of application.

It is an object of the present invention to provide a bio-re-absorbable bone adhesive with the following qualities: good biocompatibility, good wet adhesion, degradation tailored to promote healing, initially low strength within 24 hours of application. It is a further objection of the invention to provide an adhesive for sternal closure that does not have a high bonding strength within 24 hours for emergency treatment convenience, but it should be enhanced over time, up to a certain strength to resist fracture after 1-2 days and reach a very high strength in a week's time. Degradation should be inversely proportional to healing within 3-4 months.

It is an object of the invention to provide a suitable, biodegradable bone adhesive tailored to the specific requirements of this application that can allow for easy access within 24 hours of the operation.

### Statements of the Invention

Accordingly to the present invention as set out in the appended claims, there is provided a dendritic or hyperbranched polymer comprising a plurality of branches, wherein at least one of the branches comprises at least one catechol moiety that is oxidisible to a semiquinone moiety and/or a quinone moiety.

It is preferred that the dendritic or hyperbranched polymer of the invention is bioresorbable, biodegradable, and/or bioabsorbable.

Suitably, in the dendritic or hyperbranched polymer of the invention the catechol moiety is derived from a DOPA moiety or dopamine moiety or substituted phenethylamine. It will be understood that a DOPA moiety is one based on dihydroxyphenylalanine (DOPA), which can be unsubstituted or substituted with at least one other functional group. It will also be understood that a dopamine moiety means dopamine (DA) that can be unsubstitued or substituted with at least one other functional groups, such as amine groups.

Desirbly, in the dendritic or hyperbranched polymer of the invention, each of the plurality of polymeric branches may comprise an alkyl based chain from which further alkyl based chains split off to form further branches.

Suitably, in the dendritic or hyperbranched polymer of the invention, the dendritic or hyperbranched polymer may comprise at least one other functional group.

Suitably, the at least one other functional group may be selected from the group consisting of: a vinyl functionality which may an unsubstituted vinyl or a functionalized vinyl group, an amine functionality, a disulfide functionality, at least one ionisable amine, diamine, tri-amine, amino alcohol functionality, and/or combinations thereof. Desirable, the amine group may be a primary, a secondary or a tertiary amine group or a diamine group of general formula -NH-alkyl-NH₂, wherein the alkyl group is a linear or branched alkyl group having from 1 to 6 carbon atoms, and which can be substituted or unsubstituted with C₁₋₆ alkyl. Desirably, the amine group or diamine groups comprise cationic amine groups or amine groups that become cationic under certain conditions, i.e., ionisable. Suitably such conditions include physiological conditions. Particularly preferred are tertiary amines, as they are easily protonated at acidic pH.

In a particularly preferred embodiment, in the polymer of the invention the terminating vinyl group may be provided as a methacrylate group which can be unsubstituted or substituted with C₁₋₆ alkyl. The vinyl group may also be provided as C₁- C₆ alkylmethacrylate, for example, methyl, ethyl propyl, butyl, pentyl or hexyl methacrylate group. The alkyl groups in these instances may be substituted or unsubstituted with at least one methyl or ethyl group.

Preferably, at least one co-vinyl monomers or trimer may be used in the process of the invention for copolymerisation with the catechol moiety and may have the general structure: 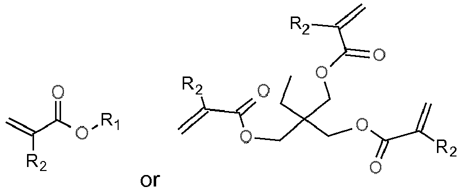 wherein R₁ is C₁- C₆ alkyl-amine selected from the group consisting of: C₀- C₆ alkyl-NH₂, C₀-C₆ alkyl-NHR₂, C₀- C₆ alkyl-NR₂ R₃, wherein independently R₂ and R₃ are -H or C₀- C₆ alkyl, and R₂ is C₀-C₆ alkyl. Particularly preferred alkyl groups are methyl, ethyl, propyl or butyl groups, which can be linear or branched. A preferred alkyl group is methyl. Preferably, R₁ is methyl, ethyl or propyl, or combinations thereof. Preferably, the amine is dimethyl or diethyl amine. Trimer type starting materials for copolymerisation are useful where dendrimers are required, whereas monomers are used where hyperbranched polymers are desired.

Suitably, particularly preferred monomer are: poly(ethylene glycol) methacrylate, N-(2-hydroxypropyl)methacrylamide., 2-dimethylaminoethyl prop-2-enoate, 2(dimethylamino ethyl) methacrylate or 2(dimethyleamino ethyl) acrylate, although other monomers having vinyl and amine groups can be used. Preferably the trimer is trimethylol-propane triacrylate (TMPTA) or Pentaerythritol triacrylate and the dimer is Poly(ethylene glycol) diacrylate, ethylene glycol diacrylate and Diethylene glycol diacrylate

Suitably, the ratios of divinyl disulfide monomer or trimer to co-vinyl monomer may be in the range of from 1:99 to 99:1. A preferred polymer with some knotting has a ration of 10:90.

In a preferred embodiment, in the dendritic or hyperbranched polymer of the invention, at least one of the plurality of polymeric branches terminates with a hydroxyl functionality. Desirably, at least one of the each of the plurality of polymeric branches terminates with a halide selected from the group consisting of: F, Cl, Br and I.

Suitably, the dendritic or hyperbranched polymer of the invention is polydisperse and has a relative high polydispersity index meaning that there will be a lot of molecular weight enhancement when go crosslinking as determined by gel permeation chromatography (GPC).

In a preferred embodiment, the alkyl based chain of the polymer may be an ethylene chain which may be unsubstituted or substituted with a linear or branched C₁₋₆ alkyl. Unsubstituted ethylene chains are particularly preferred.

In a particularly preferred embodiment, the polymer of the invention is hyperbranched and comprises a plurality of branched alkylene chains, wherein at least one of the alkylene branches comprises at least one DOPA moiety or dopamine moiety in the alkylene chain, and the alkylene sidechain is further substituted with at least one vinyl group or vinyl functionality; and wherein at least one of the alkylene branches terminates with at least one of a carbocation, a halide, an ionisable amine, diamine and a hydroxyl group.

In a preferred embodiment there is provided a polymer comprising at least one polymeric ethylene backbone having: at least one first sidechain repeating unit, wherein one end of each sidechain is covalently bonded to the ethylene backbone by an ester, amide or thioate linkage, and wherein the other end of each first sidechain comprises a catechol moiety, such as DOPA moiety or dopamine moiety and at least one of a vinyl group, a hydroxide, a halide, an amine group or a diamine group to form a polymer or comprises at least one an ester, amide or thioate linkage to a polymeric ethylene backbone (which can be same or different) to form a branched polymer having at least two polymeric ethylene backbones, and wherein each of the first sidechains further comprises at least one disulfide group located between either ends of the first sidechain repeating unit; and at least one second sidechain repeating unit, wherein one end of each sidechain is covalently bonded to the at least one polymer ethylene backbone by an ester, amide or thioate linkage, and wherein the other end of each second sidechain comprises with an ionisable amine or a diamine group.

In a preferred embodiment, the polymer further comprises a plurality of additional polymer backbone chains having the at least one first sidechain repeating unit and the at least one second sidechain repeating unit as defined above, the plurality of additional polymer backbone chains being arranged in a branched polymeric repeating pattern to form a hyperbranched polymer.

Thus the invention provides a linear, branched or hyperbranched polymer having controlled molecular weights and narrow molecular weight distributions. Suitably, the molecular weight, and the composition (i.e. the number and ratio) of first and second sidearm chains can be controlled or adjusted by appropriate choice of the co-monomer composition used to provide the polymer (for example, by a Michael Addition, click chemistry, RAFT, ATRP or DE-ATRP or other controlled living polymerization process). Desirably, quaternisation reaction of the polymer with reagents such as methyl iodide can leads to water-soluble cationic polyelectrolytes with linear, branched or hyperbranched structures. The polymers of the invention are a significant achievement, as it is known in the art that synthesis of well-defined branched polymers with ionic or ionisable groups is difficult.

Preferably, the polymer of the invention is a knotted polymer which can be single cyclized or multi-cyclized.

Desirably, in the dendritic or hyperbranched polymer of the invention the vinyl group or vinyl functionality is a methacrylate group which can be unsubstituted or substituted with a linear or branched C₁₋₆ alkyl. Methyl and ethyl substituents are preferred.

Suitably, at least one of the plurality of polymeric branches comprises at least one of an ester, ether, thioate or amide functionality.

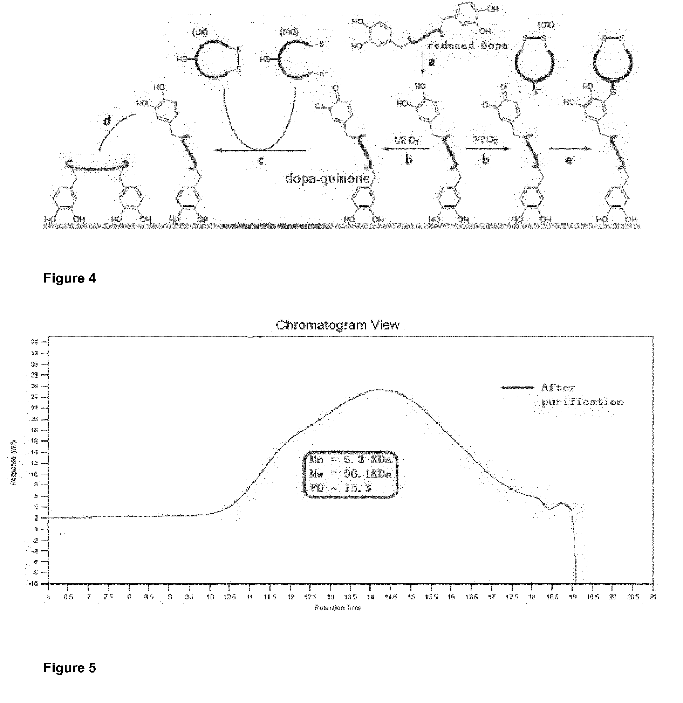

In a particularly preferred embodiment, of the invention, there is provided a dendritic or hyperbranched polymer having the ¹H NMR spectrum shown in Figure 5.

Suitably, the polymer of the invention may be derived from co-polymerisation of dopamine HCl monomer and trimethylol propane triacrylate monomer (TMPTA).

Suitably, the polymer is poly-dopamine-co-acrylate.

In a related aspect of the invention, in the dendritic or hyperbranched polymer of the invention, at least one of the plurality of branches may be crosslinked with a crosslinking agent or crosslinker system, which can optionally be used with a crosslinking promoter. Suitably, the branches may be crosslinked with chitosan or a crosslinker system that is promoted by oxidation. Desirably, the crosslinker system is permanganate, Fe(III), fibrinogen, HPR and peroxide, or combinations thereof. The Fe(III) may be provided in the form of FeCl₃, Fe(acac)₃ or other Fe(III) salt.

In a related aspect still, the polymer of the invention or a composition of a polymer of the invention may be used as medical adhesive or bone cement. Suitably, the medical adhesive is an adhesive for skin incision and/or internal wound closure. In a related embodiment, the bone cement is a cement for sternum bone closure or other orthopaedic operating procedure. Accordingly, the polymer of the invention may be used in the manufacture of a medical adhesive or a bone cement.

In a further related aspect still, there is provided a process for synthesising a dendritic or hyperbranched polymer comprising at least one catechol moiety by polymerising a divinyl monomer or a triacrylate with other optional polymerisable monomers and/or initiators including those having at least one catechol moiety under suitable polymerisation conditions to effect polymerisation of the monomer by a Michael Addition, a click chemistry or a living polymerisation process.

### Brief Description of the Drawings

Figure 1 shows synthetic mechanism and strategy for the catechol-modified dendritic polymer adhesive of the invention. The target polymeric adhesive was prepared via a one step Michael addition or click chemistry procedure;

Figure 2 shows synthesis route and schematic structure of poly-dopamine-co-triacrylate, Michael addition;

Figure 3 shows crosslinking mechanism of PDA with Fe³⁺;

Figure 4 shows the Redox control and the stepwise adsorption and cross-linking of DOPA residues with multi-thiol groups as crosslinker.

Figure 5 shows a GPC trace for the purified poly-dopamine-co-acrylate (PDA);

Figure 6 shows ¹H NMR trace of poly-dopamine-co-acrylate (PDA);

Figure 7 shows shear-lap test result of our developed poly-dopamine-co-acrylate (PDA) with three different kinds of crosslinker with fibrin glue as control. The results indicate fibrin glue is very weak and is not strong even enough to be tested within 1 h curing time. The poly-dopamine-co-acrylate (PDA) of the invention can reach a much higher adhesion strength in a short time (15 mins);

Figure 8 shows preliminary PMMA shear-lap test (A) results of commercially-available bone adhesive product Kryptonite^{™} (B) and the poly-dopamine-co-acrylate (PDA) adhesive of the invention (C). The results indicate Kryptonite^{™} hardens immediately (strength>900 KPa) which could delay emergency operation in the case of post-operative complications. The strength of the poly-dopamine-co-acrylate (PDA) adhesive of the invention was gradually increases with time and finally reach 760 KPa at 7 days; and

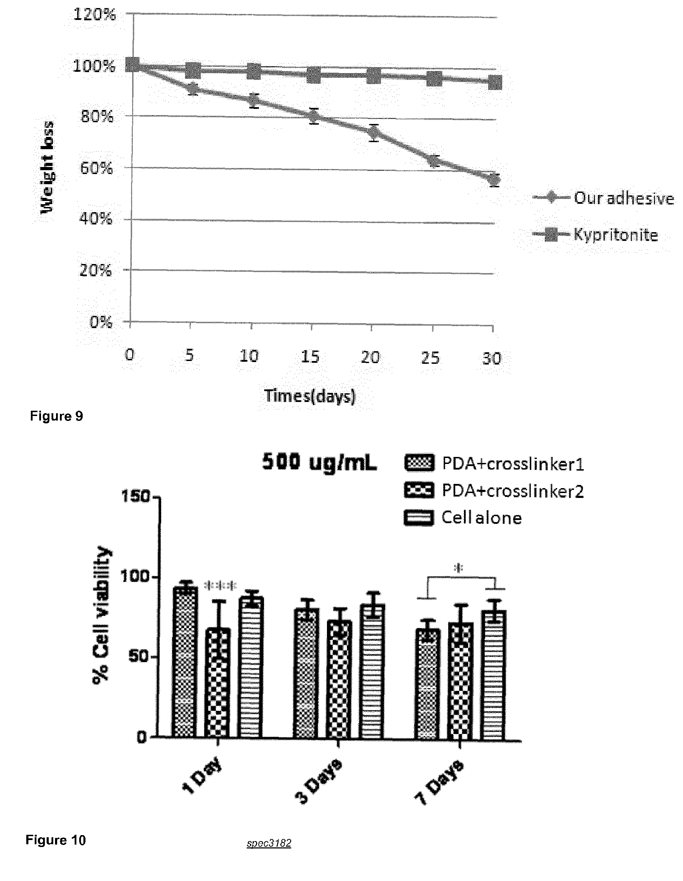

Figure 9 shows weight loss along time. Poly-dopamine-co-acrylate (PDA) adhesive of the invention shows appropriate degradable performance and degrade to almost 60% in one month time. Kryptonite hardly degrades in a one month period;

Figure 10 shows the percentage of Cell Viability in the presence of 500 µg poly-dopamine-co-acrylate (PDA) with 16µl crosslinker 1 or crosslinker 2 per mL of medium. Live/Dead staining was used to assess viability: 4 µM of Calcein AM and 1 µM of Ethidium homodimer. At 7 days, viability is not significantly reduced in the presence of PDA relatively to cells alone. * P<0.05. *** P<0.001.

### Detailed Description of the Invention

The invention pertains to the development of an adhesive for application in repairing the sternum following a median sternotomy and also for topical skin closures or for use in other orthopaedic operating treatments, such as shoulder, elbow, spine, hip, knee, wrist, foot and ankle surgery. The inventors have developed a catechol modified dendritic polymer, which achieves this goal. The adhesive of the invention is biocompatible and safe for internal use. It has wet adhesive qualities. It has a controllable curing speed with the help of different crosslinkers to allow for emergency operation within the first 24 hours and also skin closure in a short time (15 mins). Using crosslinkers (KMnO₄, Fe³⁺, HRP&H₂O₂and fibrinogen) the crosslink rate can be tailored, as well as a number of other qualities, to achieve this. For sternum bone closure, after the first couple of days the strength of the adhesive must increase to securely fixate the sternum for repair, and must achieve this strength without excessive heat production, which would cause necrosis to the surrounding tissue. Following the healing period the adhesive must degrade into non-toxic/carcinogenic/teratogenic by products that can be safely metabolised in the body.

Preliminary tests performed have already shown that for skin closure, the adhesives displays high adhesion strength in 15mins which is enough to close the wound. For sternum bone closure, the polymerisation rate over the first few days can be controlled and the adhesive of the invention shows excellent adhesive properties to the bone samples. The by-products of the degradation process of the adhesive of the invention polymer also fulfil the biocompatible necessities outlined earlier.

There are many requirements for a medical bone adhesive to clinical application in trauma and orthopaedic surgery: The adhesive and its degradation products must be non-toxic, non-carcinogenic, and non-teratogenic. The adhesive must be biocompatible to bone and surrounding tissue (no tissue irritation or tissue necrosis) with minimal heat development during polymerization. It should be biodegradable in a prefixed time - by degradation and cellular resorption - without acting as a barrier to callus formation (osteogenesis). The degradation process should act inversely proportional to healing, to ensure mechanical stability. The adhesive must bond in moist environment (high bonding strength in situ) to allow early weight bearing, needs sufficient elasticity to ensure stability to tensile strength. Easy preparation, practicability and applicability, minimal compression of volume, stability during storage, sterility and efficiency of bone adhesive. For this specific application the adhesive will also need an initially slow polymerization to allow for quick access in case of emergency operation within the first 24 hours.

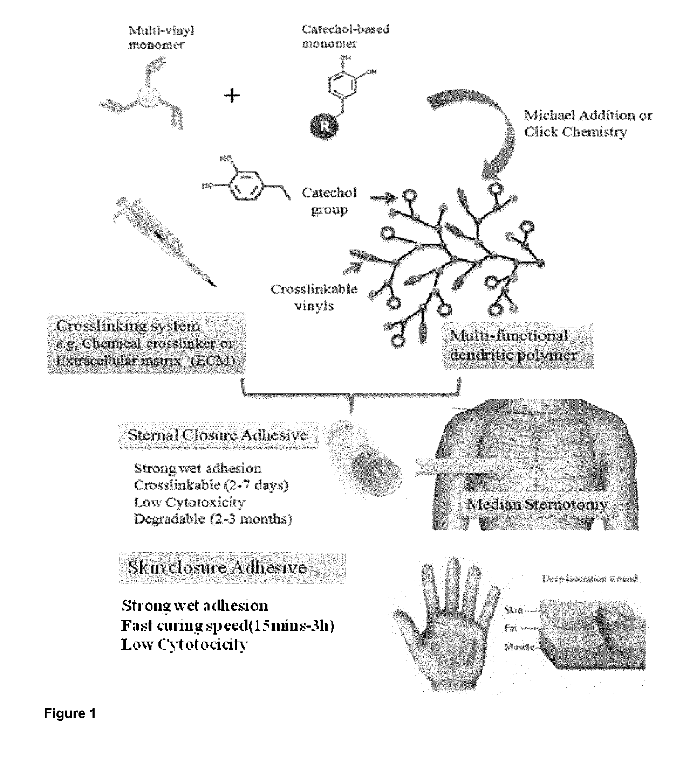

This invention focuses on developing catechol-modified dendritic polymer adhesives to overcome the drawbacks of current tissue adhesives using their unique structures and functionalities (Figure 1). This strategy has been successfully applied on incisional skin wounds in the inventors group and has shown very promising results. The successful outcome will result in major advances in healthcare - most notably for treatment of internal wound and surgical incisions wound, but also for other wound healing treatments in the future. These exciting results lead us to extend this new approach for bone adhesive application. The preliminary test results demonstrated that the pilot catechol-modified polymer that was developed showed excellent adhesion and mechanical properties for the bone model. Even more exciting is the adhesive strength can be controlled within the first week to allow easy access within the first day of bonding and gaining strength throughout the week. PDA was selected for this project as it contains dopa structure, which allows for wet adhesion, and also the functional groups present in PDA allow for tailoring of the mechanical properties.

In this study, the development of novel biodegradable and crosslinkable dendritic polymers, poly-dopamine-co-acrylate (PDA) was targeted as wet tissue adhesive materials, for example, adhesives for internal use. PDA can easily crosslink with a variety of different crosslinkers, so that the adhesive of the invention can either work as strong, secure and fast curing adhesive for topical wound closure or as controllable curing adhesives for sternum bone closure cases.

Thus, the present inventors have designed and synthesized bio-inspired dendritic polymers for wet tissue adhesive with superior properties over the current tissue adhesives by using state of the art Deactivation-Enhanced ATRP (DE-ATRP) synthetic approach.

### Synthesis of poly-dopamine-co-acrylate PDA

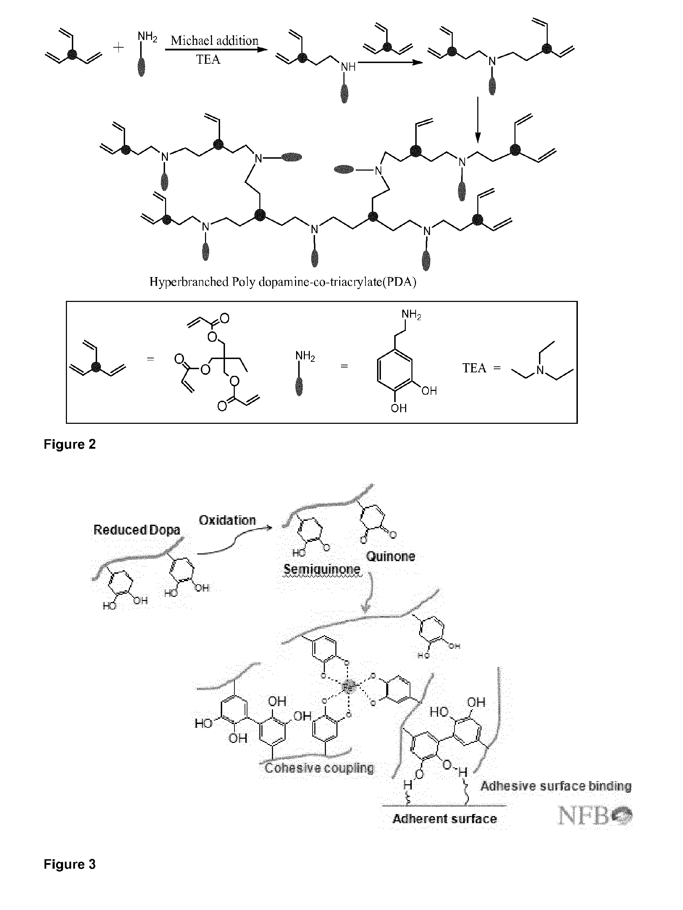

Dendritic/hyperbranched polymer poly-dopamine-co-acrylate (PDA) was successfully synthesised through the self-condensation of dopamine and trimethylol-propane triacrylate (TMPTA) monomer by Michael additions to obtain high molecular weight polymers (around 90.0kDa) (Figure 2). The targeted dendritic polymers poly-dopamine-co-acrylate (PDA) consists of catechol, vinyl and hydroxyl functional groups, leading to tailorable functionality, crosslinking (with crosslinker), biodegradability, low cytotoxicity and desired mechanical properties on dry/wet tissue.

To control the crosslinking speed it is important to understand how catechol/dopamine adhesive systems achieves adhesion. Transition metals play an integral role in the generation of a non-crystalline biological material. Oxidation of dopamine by one electron to a semiquinone occurs at a potential similar to Fe³⁺ to Fe²⁺ reduction. Semiquinone is a kind of radical which can bring about polymer-polymer coupling for cohesive bonding. It is believes that the radicals also couple directly to surfaces for adhesive interactions. Furthermore, quinone can chelate with iron to lead another way of crosslinking. We propose that the iron centre in cross-link three DOPA residues (Figure 3). With these latest observations, it appears that dopamine plays two roles in catechol/dopamine adhesive systems. Adhesive bonding to surfaces is derived from the reduced form. Cohesion in the bulk is provided by the one-electron (semiquinone) or two-electron (quinone) oxidized forms of dopamine. PDA has a tailored crosslinking speed by utilising crosslinker (KMn0₄ , Fe³⁺ , HRP/H₂O₂ or denatured fibrinogen). With KMnO₄, Fe³⁺, HRP/H₂O₂ as crosslinker, it gives a slow bonding speed. But when using denatured- fibrinogen which has multi-thiol groups as crosslinker, it achieves a faster bonding speed (Figure 4), as the oxidation of unadsorbed dopa to dopa-quinone (b) is counteracted by reducing thiolates (c), which enables enhanced adsorption (d) and depletion of thiolate pairs in oxidized forms transforms itself into a cross-linker with reduced dopa.

### Poly-dopamine-co-acrylate(PDA) synthesis - Fabrication protocol for Poly Dopamine-co-acrylate(PDA)

Trimethylolpropane triacrylate monomer(2.96 g, 10 mmol), Dopamine hydrochloride monomer(1.90g, 10 mmol) and DMSO(10 g) were added into the two neck flask. 60µL inhibitor(4-Methoxy-benzaldehyde) was added into the mixture to get rid of the possibility of free radical polymerization.The mixtures were stirred until a clear solution formed. Oxygen was removed by bubbling argon through the solution for 10 mins at room temperature. TEA (1.42 g, 14 mmol) was added at last when going through bubbling argon and magnetically stirred for 5 mins more. The solution was stirred at 600 rpm and the polymerization was conducted at 60deg C for 2 h in an oil bath. Take GPC sample every one hour.

### Purification protocol for Poly Dopamine-co-acrylate(PDA)

40 ml THF was added into the polymer mixture and then magnetically stirred it until it was completely blended. As the TEA saline is insoluble in THF, the mixture solution went cloudy. The surplus TEA saline was separated by centrifugalization(5 mins, 4000 rpm). The clear polymer solution was added dropwise into a large amount of precipitation agent (hexane:Ethanol =1:1) (500 mL) with 100 µL inhibitor(4- Methoxy-benzaldehyde) under magnetically stirring condition. The precipitate system was left still for 0.5 h and then the upper clear liquid was poured out. Acetone was used to dissolve the polymer at the bottom of the beaker to make a 50 wt% solution.

### GPC results of Poly Dopamine-co-acrylate(PDA)

The GPC trace for the purified Poly Dopamine-co-acrylate (PDA) shown in Figure 4.

### Optimising Crosslinker

Make 2 mmol/L, 20 mmol/L and 200 mmol/L FeCl₃/H₂O solution. Make 2 mmol/L, 20 mmol/L and 200 mmol/L Fe(acac)₃/C₂H₅OH solution. Make 2 mmol/L, 20 mmol/L and 200 mmol/L KMnO₄/H₂O solution Make 0.06 wt% HRP & H₂O₂ and 0.96 wt% HRP & H₂O₂. Make 20 mg/mL denatured fibrinogen solution. A chitosan solution of 1.6 w/v% was prepared by adding 1.6 g of chitosan to 100 mL of water and intermittently adding HCl to maintain the pH at 2-3. After the mixture was stirred for 24 h, undissolved material was removed from the chitosan solution by vacuum filtration. Before conducting the crosslinking reaction, the pH of the chitosan solutions was adjusted to 5.8-6.0 using small amounts of 2 M NaOH solution.

### Mechanical Test for PDA -Qualifying adhesion strength on porcine skin

Shear-lap test result of our developed poly dopamine-co-acrylate with three different kinds of crosslinker with fibrin glue as control. The results indicate fibrin glue is very weak even is not strong enough to be tested within 1 h curing time. PDA can reach a much higher adhesion strength in a short time (15 mins).

### Qualifying adhesion strength on PMMA sheets

Heat bottles of phosphate buffered saline (PBS) in oven, for 15-20 minutes, to 37°C. Prepare samples of adhesive on PMMA using the standard method. Place samples in PBS and replace in oven for time point to allow for degradation. Remove from PBS and test mechanical strength of samples. Compare to non-degraded samples for control.

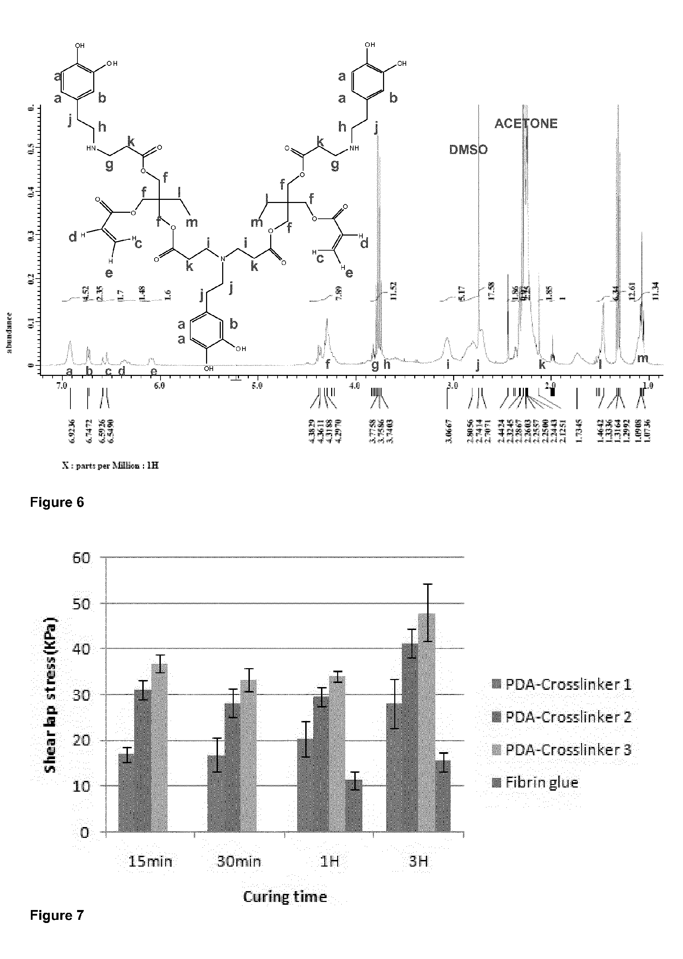

Preliminary PMMA shear-lap test (A) result of commercially-available bone adhesive product Kryptonite^{™} (B) and our developed polydopamine-co-acrylate (C). The results indicate Kryptonite^{™} hardens immediately (strength>900 KPa) which could delay emergency operation in the case of post-operative complications. The strength of PDA was gradually increases with time and finally reach 760 KPa at 7 days (see Figure 7).

### Quantifying Degradation (Weight loss)

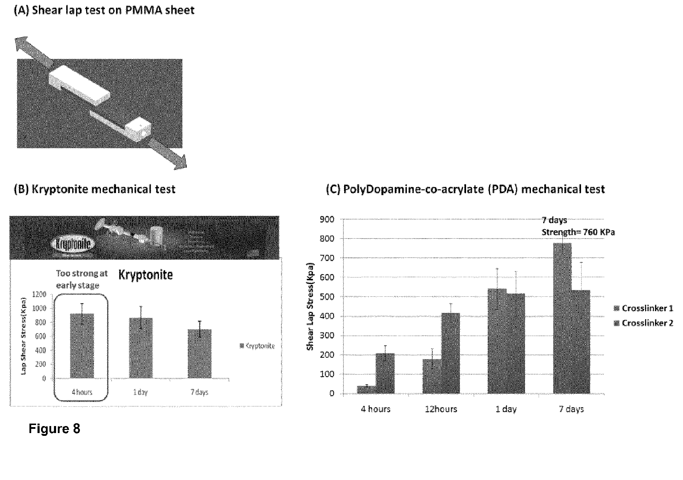

Weigh samples before placing in PBS. Heat bottles of PBS in oven, for 15-20 minutes, to 37°C. Place samples in PBS and replace in oven for time point to allow for degradation. Remove from PBS and test mechanical strength of samples. Compare to non-degraded samples for control. Weight loss along time. PDA shows appropriate degradable performance and degrade to almost 60% in one month time. Kryptonite almost do not degrade in one month time (Figure 8).

### Quantifying Cytoxicity

50,000 3T3 fibroblasts were seeded on 24 wellplates. 500 µg of PDA at a specified concentration was added for every mL of Dulbeco's Modified Eagles Medium (DMEM) used. The adhesive was added in the cell culture or directly added to the medium. Cells seeded by themselves or with acetone (same amount as in adhesive) were used as the controls. Alamar Blue and Live Dead Assay were performed after 1, 3 and 7 days. Percentage of Cell Viability in the presence of 500 µg PDA with 16µl crosslinker 1 or crosslinker 2 per mL of medium (CL1 is FeCl₃ and CL2 is KMnO₄). Live/Dead staining was used to assess viability: 4 µM of Calcein AM and 1 µM of Ethidium homodimer. At 7 days, viability is not significantly reduced in the presence of PDA relatively to cells alone. * P<0.05. *** P<0.001 (Figure 8).

Hyperbranched polymers belong to a class of synthetic tree-like macromolecules called dendritic polymers. They are polymers with densely branched structure and a large number of end groups. Dendritic polymers include dendrimers which have completely branched star-like topologies and hyperbranched polymers which have imperfectly branched or irregular structures. Both dendrimer and hyperbranched polymer molecules are composed of repeating units emanating from a central core. The core is characterised by its functionality, which is the number of chemical bonds through which it can be connected to the external parts of the molecule. The functionality of the core is normally three (e.g. amine) or four (e.g. ethylenediamine). Through the bonds of the core, the layers of linear units (single monomers or linear chains) are attached to the core and each of these arms is terminated with the multifunctional branched unit. Larger molecules are created by adding shells of linear units to the end groups of the layer beneath. If all of these units are attached to the molecule perfectly, a dendrimer is formed. In contrast, the absence of any of these units in the molecule will result in a hyperbranched polymer structure.

Furthermore, the invention provides a synthesis platform suitable for the production of specifically designed adhesives that are demonstrated to deliver improved adhesive properties within the context of the invention. The methods provided herein are capable of synthesizing polymers with impressive biodegradable properties [intracellular] and the specific addition of modifiable functional groups, making attachment of moieties of a variety of other applications attachment a genuine prospect.

In particular, the Inventors have demonstrated the synthesis and characterisation of hyperbranched or linearly branched cationic polymers suitable for use as medical adhesives, and/or bone cements.

Polymer Synthesis & Characterisation -Copolycondensation reactions are not suitable for forming hyperbranced copolymers as vinyl monomers cannot be polymerized by these methods. However, SCVP can be used to produce such hyperbranched polymers. Typically, a vinyl monomer of structure AB can be used, where A is a vinyl group and B is a functional group that can be converted into an initiating group B* by an external stimulus. This species is known as an inimer. The activated species can be a "living" free radical, an electrophilic cationic moiety or a carbanion. Inimers often comprise halogen groups, whereby activation occurs by removing the halogen to form either a cation or a radical. Controlled/living radical polymerisation (CRP) is a widely used technique that allows the synthesis of defined polymer architectures through precise control of molecular weights and distributions. The architectures of polymers prepared by the CRP techniques are reported to be the linear, crosslinked, branched/dendritic structures. By precisely controlling the competition between chain growth and reversible chain termination, the polymers can grow slowly and effectively while branching is introduced by the multi-functional vinyl monomers in a controlled fashion and the cross linking reaction is delayed. DE-ATRP allows the simple use of readily available and inexpensive multi-functional vinyl momomers to synthesis soluble hyperbranced polymers with a high degree of branching and controlled chain structure via homo- and copolymerisations. The key to the strategy is to control competition between chain growth and revesible chain termination via a deactivation enhanced method. Thus the polymer chains can grow effectively and brancing is introduced by multifunctional vinyl monomer in a controlled fashion, leading to the formation of hyperbranced polymers rather than cross linked gel.

The adhesives of the invention are synthetic polymers that are synthesised by the process of Atom Transfer Radical Polymerisation (ATRP) or Reversible Addition Fragmentation Chain-transfer (RAFT) polymerization to yield hyperbranched or linearly branched polymers. Other synthetic possibilities include Michael Addition and click chemistry processes.

## Claims

1. A dendritic or hyperbranched polymer comprising a plurality of branches, wherein at least one of the branches comprises at least one catechol moiety that is oxidisible to a semiquinone moiety and/or a quinine moiety.

2. A dendritic or hyperbranched polymer according to claim 1 wherein the catechol moiety is derived from DOPA or dopamine.

3. A dendritic or hyperbranched polymer according to claim 1 or claim 2 comprising at least one other functional group selected from the group consisting of: a vinyl functionality which may an unsubstituted vinyl or a functionalized vinyl group, an amine functionality, a disulfide functionality, at least one ionisable amine, diamine, tri-amine, amino alcohol functionality, and/or combinations thereof and wherein optionally at least one of the plurality of polymeric branches terminates with a hydroxyl functionality or terminates with a halide selected from the group consisting of: F, Cl, Br and I.

4. A dendritic or hyperbranched polymer according to any preceding claim in which the dendritic or hyperbranched polymer is polydisperse and optionally is single cyclized or multi-cyclized.

5. A dendritic or hyperbranched polymer according to any one of claims 3 to 5 wherein the vinyl group or vinyl functionality is a methacrylate group which can be unsubstituted or substituted with a linear or branched C₁₋₆ alkyl.

6. A dendritic or hyperbranched polymer according to claim 1 wherein the polymer is derived from co-polymerisation of dopamine HCl monomer and trimethylol propane triacrylate monomer (TMPTA) and has the ¹H NMR spectrum shown in Figure 5.

7. A dendritic or hyperbranched polymer according to claim 1 wherein the polymer is poly-dopamine-co-acrylate.

8. A dendritic or hyperbranched polymer according to any preceding claim, wherein at least one of the plurality of branches are crosslinked with a crosslinking agent or crosslinker system, which can optionally be used with a crosslinking promoter.

9. A dendritic or hyperbranched polymer according to claim 8 wherein the branches are crosslinked with fibrinogen, chitosan or a crosslinker system that is promoted by an oxidation reaction.

10. A dendritic or hyperbranched polymer according to claim 9 wherein the oxidation promoted crosslinker system is permanganate, Fe(III), HPR (Horseradish peroxidase) and peroxide, or combinations thereof.

11. Polymer according to any preceding claim or a composition of a polymer according to any preceding claim, for use as medical adhesive or bone cement.

12. Polymer or composition for use according to claim 11 wherein the bone cement is a cement for sternum bone closure or other orthopaedic operating procedure.

13. Use of a polymer according to any one of claims 1 to 12 in the manufacture of a medical adhesive.

14. A process for synthesising a dendritic or hyperbranched polymer comprising at least one catechol moiety by polymerising a divinyl monomer or a triacrylate with other optional polymerisable monomers and/or initiators including those having at least one catechol moiety under suitable polymerisation conditions to effect polymerisation of the monomer by a Michael Addition, a click chemistry or a living polymerisation process.

15. A method of curing an adhesive or bone cement comprising a polymer as defined in any one of claims 1 to 10 comprising the step of crosslinking the plurality of polymer branches with a crosslinking system as defined in claim 8.
